# EUROPÄISCHE PATENTSCHRIFT

(11) **EP 1 382 002 B1**
(45) Veröffentlichungstag und Bekanntmachung des Hinweises auf die Patenterteilung: **09.11.2005**
(21) Anmeldenummer: 02716606.5
(22) Anmeldetag: 21.02.2002
(51) Int. Cl.: G06K 9/00, G06F 19/00, G06T 7/00, G06T 5/00, A61B 6/14, G06F 3/033, G06F 17/60, G06F 17/30

(54) **ZAHNIDENTIFIKATION AUF DIGITALEN RÖNTGENAUFNAHMEN UND ZUORDNUNG VON INFORMATIONEN ZU DIGITALEN RÖNTGENAUFNAHMEN**
TOOTH IDENTIFICATION ON DIGITAL X-RAY PICTURES AND THE ASSIGNMENT OF INFORMATION TO DIGITAL X-RAY PICTURES
IDENTIFICATION DE DENTS SUR RADIOGRAPHIES NUMERIQUES ET AFFECTATION D'INFORMATIONS A DES RADIOGRAPHIES NUMERIQUES

(30) Priorität: 21.02.2001 DE 10108295
(43) Veröffentlichungstag der Anmeldung: 21.01.2004
(60) Teilanmeldung: 05107181.9
(73) Patentinhaber: Sirona Dental Systems GmbH, 64625 Bensheim (DE)
(72) Erfinder: ZIMMERMANN, Jürgen, 64585 Biebesheim (DE)
(74) Vertreter: Sommer, Peter
(86) Internationale Anmeldenummer: PCT/DE2002/000634
(87) Internationale Veröffentlichungsnummer: WO 2002/067186

(56) Entgegenhaltungen:
- EP-A- 0 487 110
- EP-A- 0 757 309
- EP-A- 0 973 116
- WO-A-01/46895
- DE-A- 19 941 668
- US-A- 5 742 700
- STUART C. WHITE ET AL.: "Digital Imaging: A Vision for the Future" INTERNET PUBLICATION OF THE SECTION OF ORAL RADIOLOGY, [Online] 29. Januar 1999 (1999-01-29), Seiten 1-4, XP002206372 UCLA School of Dentistry Gefunden im Internet: <URL:http://www.dent.ucla.edu/sod/depts/or al_rad/VisionStatement.html> [gefunden am 2002-07-16]
- BURDEA G C ET AL: "Real Time Tooth Position Measurements For Digital Dental Subtraction Radiography" ENGINEERING IN MEDICINE AND BIOLOGY SOCIETY, 1990., PROCEEDINGS OF THE TWELFTH ANNUAL INTERNATIONAL CONFERENCE OF THE IEEE PHILADELPHIA, PA, USA 1-4 NOV. 1990, NEW YORK, NY, USA,IEEE, US, 1. November 1990 (1990-11-01), Seiten 2078-2079, XP010036401 ISBN: 0-87942-559-8

## Beschreibung

### Technisches Gebiet

Die Erfindung betriff ein Verfahren und eine Anordnung zur Identifikation von Objekten, insbesondere Zähnen, auf einer digitalisierten Röntgenaufnahme. Ein weitere Bestandteil der Erfindung ist ein Verfahren und eine Anordnung zur Zuordnung von Informationen zu Objekten, insbesondere Zähnen, die in einer digitalisierte Röntgenaufnahme oder einer schematischen Darstellung bestimmt sind.

### Stand der Technik

Ein Röntgengerät zur Erstellung von Panorama-Schichtaufnah-men und Einzelaufnahmen hiervon ist aus der DE 35 45 509 (US 4,847,881) und der DE 35 45 493 (US 4,813,060) bekannt. Digitale Röntgenaufnahmen für Panoramaschichtaufnahmen und cephalometrische Aufnahmen sind aus der EP 0 632 994 (US 5,511,106) bekannt. Die Erstellung digitaler Intraoral-Aufnahmen mit einem Intraoralsensor sind aus der EP 0 643 901 (US 5,513,252) bekannt.

Viele Verfahren der Diagnostik beziehen sich auf einzelne Details wie z.B. einzelnen Zähne, deren Existenz, Form, Lage individuell verschieden ist. Die Diagnostik und Dokumentation wird dadurch erschwert, dass die Anwender gezwungen sind, bei weiteren Arbeiten auf nicht-individuelle, sondern allgemeine Schemata auszuweichen wie z.B. das feste Zahnschema auf dem Krankenschein.

Bei automatisierten Aufnahmeserien ist es notwendig, die zu treffenden Zähne vorher im Bedienprogramm zu spezifizieren.

Dies ist momentan entweder unzureichend oder umständlich gelöst, da es entweder nur möglich ist, ganze, fix festgelegte Zahngruppen auszuwählen, oder nur einzelnen Zähne zuzuordnen. In jedem Fall geht der reale Bezug verloren, da der Bediener die vertraute Umgebung, z.B. eine reale Gebissaufnahme, verlassen muss.

Panorama-Röntgenaufnahmen dienen dazu, schnell eine Übersicht über den Gesamtzustand des Gebisses zu erhalten. Hiervon lassen sich bereits Befunde ohne weitere, z.B. intraorale, Röntgenaufnahmen ableiten. Diese Befunde beziehen sich aber nicht auf das gesamte Bild, sondern jeweils auf ein bestimmtes dargestelltes Objekt, z.B. einen bestimmten Zahn. Damit entstehen letztlich mehrere Befunde, die sich auf ein Bild beziehen und die z.B. den einzelnen Zähnen zugeordnet sind. Deren Erfassung ist umständlich, da der Anwender i.d.R. die unmittelbare Bedienumgebung des Panoramabildes verlassen muss.

Die US5742700 offenbart Zahnerkennung auf digitalisierten Röntgenbildern, wobei die Zahnbereiche durch Kantendetektion segmentiert werden. Parameter des Röntgengerätes, die bei Erstellung der Aufnahme verwendet wurden, wie z.B. Belichtungsdaten, werden zur weiteren Bestimmung im neuronalen Klassifikator mit diesen Bereichen rechnerisch verknüpft.

Aufgabe der vorliegenden Erfindung ist daher die Verbesserung des Verfahrens durch die Einbeziehung weiterer Parameter.

### Darstellung der Erfindung

Gelöst wird diese Aufgabe durch Verfahren und Anordnungen, die die Merkmale der unabhängigen Ansprüche aufweisen.

Insbesondere durch ein Verfahren zur Identifikation von Objekten, insbesondere Zähnen, auf einer digitalisierten Röntgenaufnahme. Um eine möglichst genaue Erkennung sicherzustellen, werden mit Bildverarbeitungsalgorithmen durch Segmentierung und/oder Kantendetektion der Röntgenaufnahme die das oder die möglichen Objekte abbildende Bereiche bestimmt und werden diese Bereiche zur weiteren Bestimmung mit zur Erstellung der Röntgenaufnahme verwendeten Parametern des Röntgengerätes rechnerisch verknüpft.

Das Verfahren kann darüber hinaus so weitergebildet sein, dass die gerätespezifischen Parameter mit Parametern aus einer patientenunabhängigen Zahndatenbank verknüpft werden, um wahrscheinliche aktuelle geometrische Positionen über die das oder die möglichen Objekte abbildende Bereiche zu erhalten. Weiterhin ist es möglich, bereits vor der Verknüpfung mit den gerätespezifischen Parametern eine Zusammenfassung (Cluster-Bildung) erkannter Bereiche vorzunehmen. Darüber hinaus sind zusätzlich patientenspezifische Parameter zur weiteren Bestimmung der das oder die möglichen Objekte abbildende Bereiche rechnerisch verknüpft werden.

Bei diesen zusätzlichen Parametern handelt es sich ums Positionsdaten, Bahnkurven, Start- und Endpunkte des Röntgengerätes. Durch die Positionsdaten und Bahnkurven können Aufschlüsse darüber gegeben werden, ob es sich hierbei um einen Backenzahn oder einen Schneidezahn handelt. In Verbindung mit statistischen und stochastischen Daten kann die Größe dieses Zahns bestimmt werden, wodurch eine Mustererkennung stark vereinfacht wird. Weiterhin ist es wichtig, Informationen über die Graustufen der Aufnahme zu besitzen. Anhand dieser Informationen können Kanten besser bestimmt werden. Es ist somit möglich, flächige Zahnbereiche von anderen Bereichen zu unterscheiden.

Weiterhin können in die Berechnung Informationen des Patienten einfließen. Hinweise darauf, welche Zähne nicht mehr vorhanden sind oder welche Zähne ersetzt wurden, ermöglichen es der Mustererkennung in diesen Bereichen besonders aufwändige Erkennungsverfahren einzusetzen bzw. keine Mustererkennung durchzuführen. Weiterhin können anatomische Patientenparatmeter berücksichtigten werden. Aus Hinweisen auf die Rasse, Alter, Geschlecht, Größe, Gewicht können statistische Rückschlüsse getroffen werden auf den Kieferaufbau und die Größe und Anordnungen der Zähne.

Im Falle von Erkennungsfehlern soll der Benutzer die Möglichkeit haben interaktiv zu bestimmen, welche Bereiche einem Zahn zuzuordnen sind und welche nicht. Hiefür werden dem Benutzer Vorschläge über die erkannten Objekte gemacht, die interaktiv angepasst oder bestätigt werden können. Dem Benutzer wird ermöglicht, beispielsweise durch Polygone, die er manuell einzeichnen kann, zu bestimmen, welchen Umfang ein Objekt hat.

Die so ermittelten Informationen der Objekte werden separat in einer Datenbank abgespeichert. Die Informationen werden vorzugsweise nicht direkt in die Abbildung integriert. Aus Gründen der Effizienz wird bevorzugt, dass die Informationen über die Objekte separat abgelegt werden. Hierdurch sind andere Arten von Verwendungen für die Abbildungen weiterhin gegeben, ohne dass störende Begrenzungen auf diesen Abbildungen sichtbar sind.

Ein weiterer Bestandteil der Erfindung ist eine Anordnung zur Identifikation von Objekten, insbesondere Zähnen, auf einer digitalisierten Röntgenaufnahme. Diese Anordnung weist ein Ein-und einen Ausgabegerät zur interaktiven Steuerung auf. Bei diesen Ein- und Ausgabegeräten handelt es sich vorzugsweise um eine Tastatur oder ein Zeigegerät und einen Monitor. Mit Hilfe dieses Zeigegerät können im Korrekturfall die einzelnen Bereiche bestimmt werden, die Objekte darstellen.

Ein weiterer Bestandteil der Anordnung ist eine Bearbeitungseinheit, die Zugriff auf die digitalisierte Röntgenaufnahme und auf gerätespezifische Informationen des Röntgengerätes hat und die auf Grundlage dieser Informationen sowie durch Segmentierung und/oder Kantendetektion das Objekt auf der digitalisierten Röntgenaufnahme eingrenzt. Bei dieser Bearbeitungseinheit handelt es sich vorzugsweise um bekannte Prozessoren, die auf der Grundlage dessen bereits oben beschriebenen Verfahrens die einzelnen Objekte in der Röntgenaufnahme bestimmen. Die im Verfahren beschriebenen Vorgehensweise wird vollständige durch den Prozessor ausgeführt.

Die Bearbeitungseinheit kann darüber hinaus Zugriff auf eine patientenunabhängige Zahndatenbank und/oder auf patientenspezifische Informationen haben. Die Bearbeitungseinheit kann weiterhin Mittel zur Cluster-Bildung der nach der Segmentierung und/oder Kantendetektion voriegenden Bereiche aufweisen.

So können Mittel vorgesehen sein, mit denen dem Benutzer Vorschläge unterbreitet werden, die interaktiv angenommen, abgelehnt oder abgeändert werden können.

Durch eine Schnittstelle zum Röntgengerätes kann auf gerätespezifische Daten zugegriffen werden. Bei diesen Daten handelt es sich vorzugsweise um Positionsdaten, Bahnkurven, Start- und Endpunkte, Farbskalen und/oder Farbabgleiche des Röntgengerätes, die wie bereits oben beschrieben wurden in die Berechung einfließen.

Die anatomischen Patientenparameter wie Rasse, Alter, Geschlecht, Größe und Gewicht ersehnten in einer Datenbank abgelegt. Die Anordnung gereift auf diese Datenbank zu, und werteten sie in der bereits beschriebenen Form nach vorzugsweise statistischen Daten aus. Sollten Informationen über tatsächliche Maße und Größen von Zähnen vorliegen, so werden diese natürlich vorzugsweise berücksichtigten.

Die Anordnung ist vorzugsweise als PC ausgestaltet, und weist eine bekannte serielle, parallele, busförmigen oder netzförmige Schnittstelle zum Röntgengerät auf. Der Verfahren auf dieser Anordnung wird vorzugsweise durch eine Software Software-Programmen realisiert. Anordnung nach einem oder mehreren der vorhergehenden Ansprüche, dadurch gekennzeichnet, dass die Anordnung ein PC ist, der durch Software gesteuert wird.

Ein weiterer Bestandteil der vorliegenden Erfindung ist eine Anordnung, die Informationen erkannten Objekten zugeordnet. Dieser Anordnung kann auf den bereits oben beschriebenen Anordnungen bzw. Verfahren aufbauen. Die Anordnung kann jedoch auch separat von ihnen verwendet werden, wenn bereits Objekte vorliegen. Es handelt sich hierbei um eine Anordnung zur Zuordnung von Informationen zu Objekten. Bei diesen Objekten handelt es sich vorzugsweise um Zähne, die in digitalisierte Röntgenaufnahme bestimmt sind.

Die Anordnung weist Ein- und Ausgabegeräte zur interaktiven Steuerung der Anordnung auf. Bei diesen Geräten handelt es sich vorzugsweise um Tastaturen, Zeigegeräte und Bildschirme. In einem ersten Speicherbereich sind Röntgenaufnahme abgelegt. Hierbei handelt es sich vorzugsweise um einen Containern für eine Vielzahl von digitalisierten Röntgenaufnahmen. Zusätzlich zu diesen Röntgenaufnahmen sind Informationen abgelegt, die die Objekte in der Röntgenaufnahme kennzeichnen. Die Objektkennzeichnungsinformationen haben die Aufgabe, den flächigen oder gegebenenfalls auch den räumlichen Umfang des Objektes in der Darstellung zu bestimmen. Es handelt sich vorzugsweise um separate Datenstrukturen, die durch zwei- oder mehrdimensionale Polygone das Objekt in der Röntgenaufnahme darstellen. Diese Informationen werden beim Bildaufbau über die ursprüngliche Röntgenaufnahme gelegt. Es ist jedoch auch denkbar, dass diese Informationen in die Röntgenaufnahme selber integriert werden.

In einem zweiten Speicherbereich werden die Informationen zu den Objekten abgelegt. Bei diesen Informationen kann es sich um Textinformationen oder auch um Bildinformationen handeln. So ist denkbar, dass eine Detailaufnahme in höherer Auflösung als zusätzliche Informationen gespeichert wird. Eine andere Möglichkeit besteht darin, durch grafische Markierungen einen Hinweis darauf zugeben, in welchen Bereichen Behandlungen notwendig sind oder bereits vorgenommen wurden. Es ist ebenfalls möglich mehrere Arten von Informationen einen Objekt zuzuordnen.

Weiterhin werden Referenzen zwischen den Objekten und den Informationen gespeichert. Bei diesen Referenzen handelt es sich vorzugsweise um Pointer bzw. Index-Felder, die die logische Verbindungen zwischen den Objekten und den Informationen herstellen.

Eine Bearbeitungseinheit kontrollierten die Operationen Anlegen, Löschen und Zugriff. Die Operationen werden vorzugsweise durch den Benutzer mit Hilfe der Ein- und Ausgabegeräte gestartet. Die Bearbeitungseinheit steuert somit den Zugriff auf die unterschiedlichen Speicherbereiche.

Bei diesen Speicherbereichen handelt es sich vorzugsweise nur um logisch unterschiedliche Speicherbereiche. Er kann sich physikalisch gesehen um einen zusammenhängenden Speicherbereich handeln.

Die Verwendung eines physikalischen und logischen Speicherbereichs für die Bildinformationen, die Objektinformationen und die zusätzliche Informationen ist möglich, jedoch nicht besonders vorteilhafte, da die Flexibilität verloren geht.

Um die Arbeit mit der Anordnung zu verbessern, sind die Objekte auf dem Ausgabegerät optisch hervorgehoben. Sollten auswählbare Informationen vorliegen, so werden diese durch ein weiteres Merkmal grafisch gekennzeichnet.

Die Verwendung von Referenzen ermöglicht eine hierarchische Anordnung von Informationen. Der Benutzer kann sich somit von einer Panoramaaufnahme zu Detailaufnahmen bewegen und Informationen auf jeder Ebene hinterlegen.

Vorteilhaft ist eine Anordnung, bei der die Referenzen in Form von Verweisen (Links) entweder unmittelbar beim Objekt und/oder unmittelbar bei der Information und/oder separat verwaltet werden.

In einer bevorzugten Ausgestaltung, ist die Anordnung einen PC mit einem Bildschirme, wobei die Informationen durch KontextMenüs zugänglich sind. Dieser Kontextmenüs werden in Form von Pop-Up-Fenster dargestellt, wenn ein Objekt durch den Benutzer aktiviert wird. Über das Kontext-Menü kann der Benutzer entscheiden, ob er neue Informationen hinterlegen möchte, oder ob es sich bereits vorliegende Informationen anschauen möchte.

Die weiteren Informationen sind vorzugsweise Diagnose- und/oder Behandlungsinformationen und/oder weitere insbesondere detaillierte Röntgenaufnahmen.

In einer bevorzugten Ausführungsform weist die Anordnung eine Schnittstelle zu einem Röntgengerät auf. Über diese Schnittstelle werden vom Röntgengerät Informationen in Form von Röntgenaufnahmen an die Anordnung übertragen, die im ersten oder dritten Speicherbereich abgelegt werden. Hat der Benutzer bereits zu Beginn ein Objekt bestimmten, so kann ebenfalls sofort eine Referenz im vierten Speicherbereich zu diesem Objekt angelegt werden. Die neue Röntgenaufnahme wird sofort einer betehenden Röntgenaufnahme oder einem ausgewählten Teilbereich davon zugeordnet und mit einer entsprechenden Referenz versehen. Eine hierarchische Anordnung ist somit ohne weiteres möglich, die sich gegebenenfalls über mehrere Ebenen erstrecken kann.

Sollte ein Objekt nicht bereits bestimmt worden sein, so stellt die Anordnungsmittel bereit, die eine manuelle Auswahl eines bestimmten Bereichs auf der Röntgenaufnahme zulassen.

Die Anordnung weist vorzugsweise die Funktionalität eines Datenbanksystems auf.

Vorzugsweise werden die Objekte durch eine Anordnung und ein Verfahren erkannt, wie es bereits oben beschrieben wurde. Eine Kombination der Anordnungen ist somit denkbar.

Ein weiterer Bestandteil der vorliegenden Erfindung ist ein Verfahren zur Zuordnung von Informationen zu Objekten, insbesondere Zähnen, die in einer digitalisierte Röntgenaufnahme bestimmt sind. In einem ersten Schritt wird das digitalisierte Röntgenbild dargestellt. In einem zweiten Schritt werden die Objekte manuell oder automatisch bestimmt. Dieser Schritt ist jedoch nur dann notwendig, wenn die Objekte nicht bereits schon bestimmt sind. In einem dritten Schritt wird das Objekt ausgewählt, für das weitere Informationen gespeichert, abgerufen oder gelöscht werden sollen.

Die wohl häufigste Operation ist die Abfrageoperationen, bei der Informationen aus der Datenbank geladen werden. Hierbei wird einer Referenz gefolgt, die in Relation zum Objekt abgelegt ist, um anhand dieser Referenz die Informationen zu bestimmten, die dargestellt werden sollen.

Bei einer Löschoperation wird ebenfalls der Referenz gefolgt wird, die in Relation zum Objekt abgelegt ist. Diese Referenz und ggfs. die Information werden daraufhin gelöscht. Eine Informationen wird immer nur dann gelöscht, wenn sie lediglich von einem Objekt referenziert wird. Es kann der Fall eintreten, dass mehrere Objekte eine Information referenzieren. Wird in diesem Falle ebenfalls die Informationen gelöscht würde, so fehlt für zumindest ein Objekt die Informationen. Bei einer Speicheroperation wird ein Objekt ausgewählt und Speicherbereich für die Information bereitgestellt. Weiterhin wird ein Speicherbereich für die Referenz bereitgestellt, um dann die neuen Informationen und die entsprechend Referenz in diesen Speicherbereichen abzulegen.

Es ist ebenfalls denkbar, dass nach der Bestimmung des Objektes digitale Abbildungen vom Röntgengerät empfangen werden, die automatisch dem bestimmten Objekt zugeordnet werden. Hierbei wird ausreichend viel Speicherbereich bereitgestellt (allokiert), um die Informationen abzulegen.

In einer bevorzugten Ausgestaltung sind die Informationen grafische Markierungen, die als Overlay über die Aufnahmen gelegt werden können. Hierdurch ist es möglich Detailinformationen, die auf der Abbildungen nicht zuerkennen waren, nachträglich zu verfeinern und zu bearbeiten.

Für den Fall, dass Objekte in weitere Bereiche aufgeteilt werden sollten, stellt das Verfahren die Möglichkeit zur Verfügung, dass Bereiche der Objekte bestimmt werden können, in denen Informationen zugeordnet werden können.

Der Zugriff auf die Informationen erfolgt vorzugsweise durch Kontextmenüs, die zu den einzelnen Objekten abgerufen werden können.

Die Verfahren werden vorzugsweise durch Software realisiert, die auf einen bekannten PC abläuft. Darüber hinaus kann ein Datenträger vorgesehen sein, der eine Datenstruktur aufweist, die nach dem Ladevorgang ein Verfahren nach einem oder mehreren der vorhergehenden Verfahrensansprüche durchführt.

Weiter vorteilhafte Ausführungsformen sind in den Unteransprüchen aufgeführt.

### Kurzbeschreibung der Zeichnung

Es folgt eine detaillierte Beschreibung anhand der Zeichnungen. Es zeigt:
- Fig. 1: den schematischen Ablauf bei der Bestimmung von Zähnen;
- Fig. 2: Informationen, die zu einem Objekt (Zahn) abgelegen wurden;
- Fig. 3a, b ein Kontextmenü für ein Objekt;
- Fig. 4: Panoramaaufnahme mit einem erkannten Zahn.

### Ausführungsbeispiel

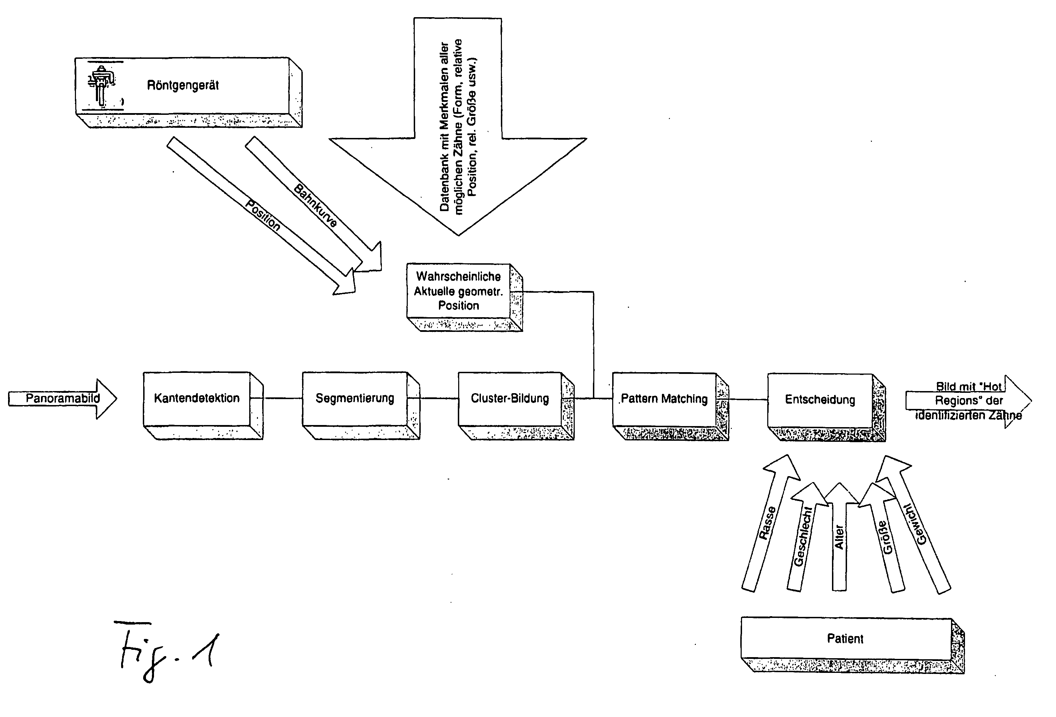

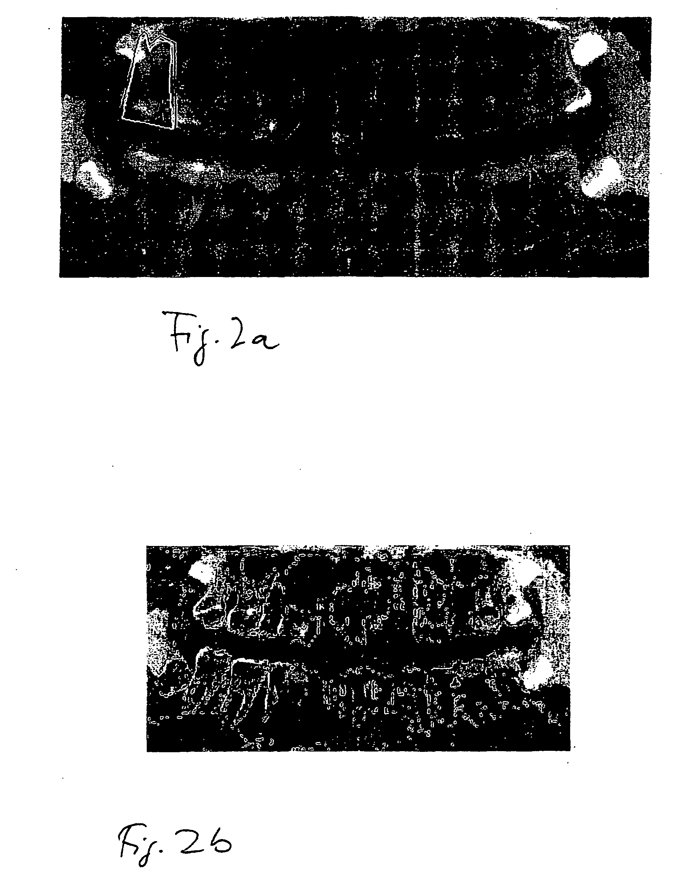

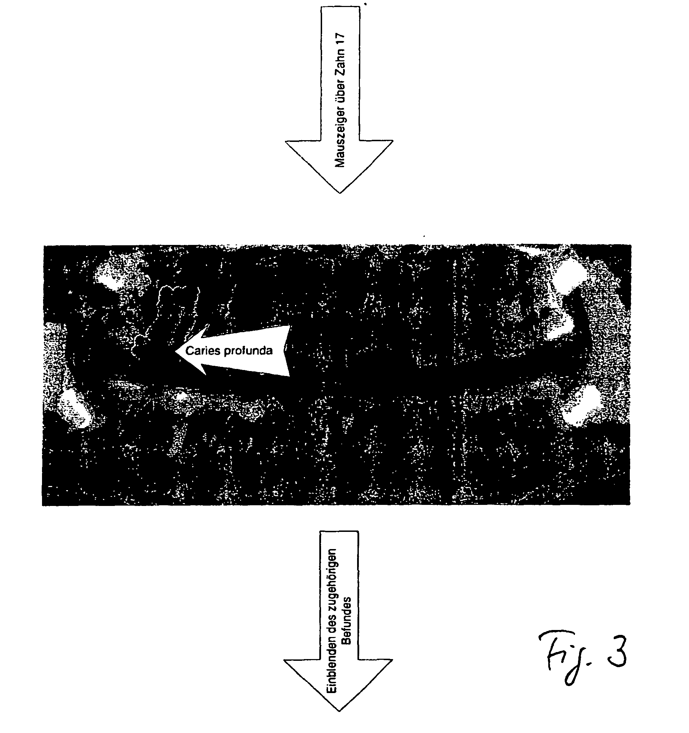

Die Fig. 1 zeigt schematisch, welche Informationen vom Röntgengerät und vom Patient nach der Bilderkennung durch Kantenfindung und Segmentierung verwendet werden können. Sollten Kanten und Segmente gefunden worden sein, so wird versucht, durch Cluster-Bildung die erkannten Bereiche und Kanten zu gruppieren. Um den entsprechenden Zahn zu identifizieren, werden unter Berücksichtigung der Geräteparameter diese Gruppen mit Zahnformen aus einer patientenunabhängigen Datenbank verglichen. Unter Berücksichtigung der patientenabhängigen Parameter wird dann eine weitere Verarbeitung durchgeführt. Als Ergebnis wird eine Röntgenaufnahme dargestellt, die durch entsprechende Regionen, d. h. Objekte, bestimmt und unterteilt ist. Diese Regionen stehen für die erkannten Zähnen. Eine solche Region ist den Fig. 2a, 2b zu entnehmen.

Die Fig. 2a zeigt einen erkannten Zahn, für den Informationen hinterlegt werden können. Der erkannte Zahn wird durch eine entsprechende Umrandung dargestellt.

In Fig. 2b ist auf der linken Bildhälfte eine Gruppe von Zähnen durch einen Mausklick ausgewählt worden und ist als Folge davon in ihrer Kontur hervorgehoben dargestellt. Es handelt sich um Zähne in der oberen und unteren Zahnreihe. Den so ausgewählten Zähnen können nun Informationen zugeordnet werden. Werden aber z.B. zu viele Zähne erfasst oder soll die Sensorpositionierung etwas verschoben werden oder sollen erfaßte Zähne keine Rolle spielen, da sie zwar zu sehen, jedoch nicht Gegenstand der Diagnose sein werden, kann eine An- bzw. Abwahl einzelner oder mehrerer Zähne durch z.B. übliche Mausaktionen wie Einfach- oder Doppelklick, Aufziehen eines Bereiches usw. direkt über den ausgewählten Bereichen des Hintergrundbildes erfolgen. Insbesondere für Aufnahmeserien ist es notwendig, die zu treffenden Zähne vorher im Bedienprogramm einzeln zu spezifizieren.

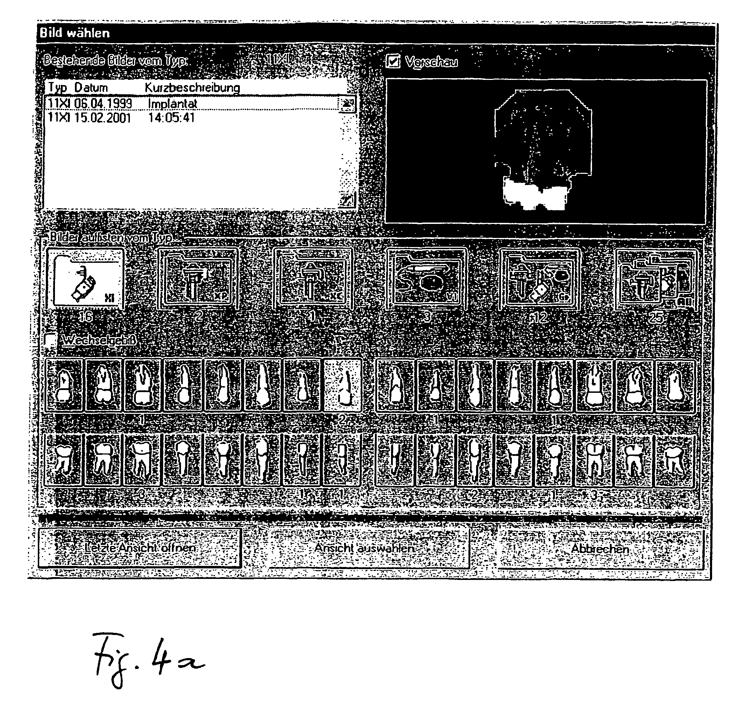

Fig. 3 zeigt eine mögliche Darstellungsform von Informationen, die Objekten zugeordnet wurden. Im vorliegenden Fall handelt es sich um Karies. Dieser Hinweis wird immer dann angezeigten, wenn der Mauszeiger über den entsprechenden Zahn gefahren wird. Andere Darstellungsformen sind jedoch auch denkbar, insbesondere mit kleinen grafischen Symbolen, die darauf hinweisen, dass weitere Informationen für dieses Objekt hinterlegt sind.

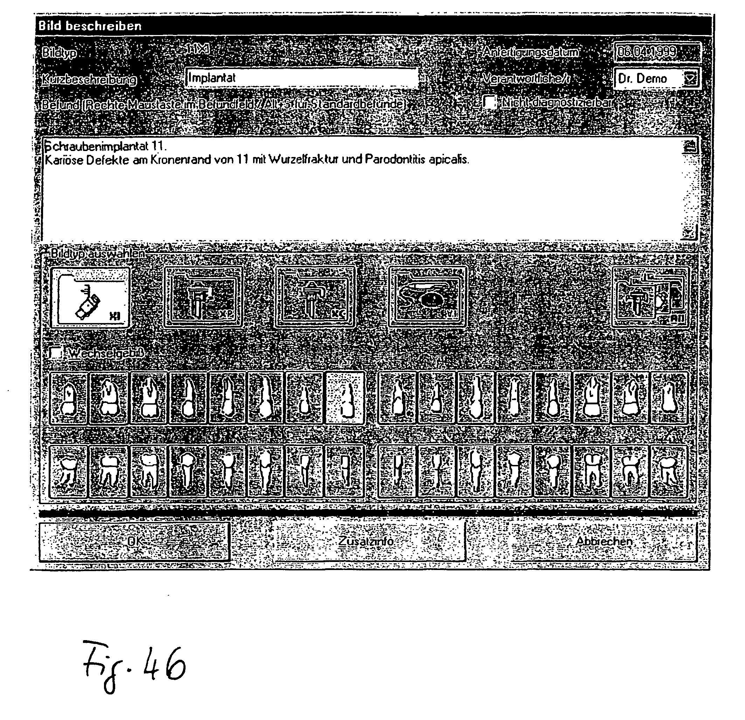

Die Figuren 4a und 4b zeigen eine mögliche Verwaltung von digitalen Röntgenaufnahmen. Hierbei wird jedem Zahn, so weit er vorhanden ist eine entsprechenden Röntgenaufnahme zugeordnet. Diese Zuordnung kann natürlich nur dann erfolgen, wenn entsprechende Aufnahmen vorliegen. Die Figuren 4a und 4b zeigen die Möglichkeit auf, dass die Objekte nicht auf einer Röntgenaufnahme abgegrenzt sein müssen, sondern auch auf einer schematischen Darstellung angeordnet sein können.

Das Verfahren kann in Form einer Software nach einem oder mehreren der nachstehenden Verfahrensansprüche niedergelegt sein. Ein Datenträger kann eine ablauffähige Datenstruktur, die auf einem Computer ein Verfahren nach einem oder mehreren der nachstehenden Verfahrensansprüche realisiert, enthalten.

## Patentansprüche

1. Verfahren zur Identifikation von einem oder mehreren Objekten, insbesondere Zähnen, auf einer digitalisierten Röntgenaufnahme, wobei mit Bildverarbeitungsalgorithmen durch Segmentierung und/oder Kantendetektion der Röntgenaufnahme die das oder die möglichen Objekte abbildenden Bereiche bestimmt werden, **dadurch gekennzeichnet, dass** diese Bereiche zur weiteren Bestimmung mit zur Erstellung der Röntgenaufnahme verwendeten Parametern des Röntgengerätes rechnerisch verknüpft werden, wobei als Parameter Positionsdaten und/oder Bahnkurven und/oder Start- und Endpunkte des Röntgengerätes in die Berechung einfließen.

2. Verfahren nach Anspruch 1, **dadurch gekennzeichnet, dass** die gerätespezifischen Parameter mit Parametern aus einer patientenunabhängigen Zahndatenbank verknüpft werden, um wahrscheinliche aktuelle geometrische Positionen über die das oder die möglichen Objekte abbildende Bereiche zu erhalten.

3. Verfahren nach Anspruch 1 oder 2, **dadurch gekennzeichnet, dass** vor der Verknüpfung mit den gerätespezifischen Parametern eine Zusammenfassung (Cluster-Bildung) erkannter Bereiche erfolgt.

4. Verfahren nach einem der Ansprüche 1 bis 3, **dadurch gekennzeichnet, dass** zusätzlich patientenspezifische Parameter zur weiteren Bestimmung der das oder die möglichen Objekte abbildende Bereiche rechnerisch verknüpft werden.

5. Verfahren nach einem der Ansprüche 1 bis 4, **dadurch gekennzeichnet, dass** anatomische Patientenparameter wie Rasse, Alter, Geschlecht, Größe, Gewicht und/oder bisherige Behandlungen in die Berechung einfließen.

6. Verfahren nach einem der Ansprüche 1 bis 5, **dadurch gekennzeichnet, dass** dem Benutzer Vorschläge über die erkannten Objekte gemacht werden, die interaktiv angepasst oder bestätigt werden können.

7. Verfahren nach einem der Ansprüche 1 bis 6, **dadurch gekennzeichnet, dass** die so ermittelten Informationen der Objekte separat in einer Datenbank abgespeichert werden und zur erneuten Verwendung abrufbar sind.

8. Verfahren nach einem der Ansprüche 1 bis 7, **dadurch gekennzeichnet, dass** das digitalisierte Röntgenbild dargestellt wird und dass das Objekt ausgewählt wird, für das weitere Informationen gespeichert werden sollen, und dass weiterhin nach der Bestimmung des Objektes von einem Röntgengerät Daten zur Darstellung von digitale Abbildungen empfangen werden, die automatisch dem ausgewählten Objekt zugeordnet werden.

9. Verfahren nach Anspruch 8, **dadurch gekennzeichnet, dass** das ausgewählte Objekt mit einer grafischen Markierung versehen ist, die als Overlay über die Aufnahmen gelegt wird.

10. Verfahren nach einem oder mehreren der Ansprüche 8 bis 9, **dadurch gekennzeichnet, dass** die Informationen grafische Markierungen sind, die als Overlay über die Aufnahmen gelegt werden können.

11. Verfahren nach einem oder mehreren der Ansprüche 8 bis 10, **dadurch gekennzeichnet, dass** Bereiche des Objekts bestimmt werden können, in denen Informationen zugeordnet werden können.

12. Verfahren nach einem oder mehreren der Ansprüche 8 bis 11, **dadurch gekennzeichnet, dass** Kontextmenüs zu den einzelnen Objekten abgerufen werden können.

13. Anordnung zur Identifikation von Objekten, insbesondere Zähnen, auf einer digitalisierten Röntgenaufnahme,
- mit einem Ein- und einem Ausgabegerät zur interaktiven Steuerung der Anordnung,
- mit einer Bearbeitungseinheit, die Zugriff auf die digitalisierte Röntgenaufnahme hat, die Zugriff auf gerätespezifische Parameter des Röntgengerätes hat und die mittels Bildverarbeitungsalgorithmen auf Grundlage dieser Parameter sowie durch Segmentierung und/oder Kantendetektion die das Objekt abbildenden Bereiche auf der digitalisierten Röntgenaufnahme bestimmt, wobei als gerätespezifische Parameter Positionsdaten und/oder Bahnkurven und/oder Start- und Endpunkte des Röntgengerätes für die Berechung abgerufen werden.

14. Anordnung nach Anspruch 13, **dadurch gekennzeichnet, dass** die Bearbeitungseinheit Zugriff auf eine patientenunabhängige Zahndatenbank hat.

15. Anordnung nach Anspruch 13 oder 14, **dadurch gekennzeichnet, dass** die Bearbeitungseinheit Mittel zur Cluster-Bildung der nach der Segmentierung und/oder Kantendetektion vorliegenden Bereiche aufweist.

16. Anordnung nach einem der Ansprüche 13 bis 15, **dadurch gekennzeichnet, dass** die Bearbeitungseinheit Zugriff auf patientenspezifische Informationen hat.

17. Anordnung nach einem der Ansprüche 13 bis 16, **dadurch gekennzeichnet, dass** Mittel vorgesehen sind, um dem Benutzer ein Vorschlag zu unterbreiten, der interaktiv angenommen, abgelehnt oder abgeändert werden kann.

18. Anordnung nach einem der Ansprüche 13 bis 17, **gekennzeichnet durch** eine Schnittstelle zum Röntgengerät, über die auf die gerätespezifischen Daten zugegriffen werden kann.

19. Anordnung nach einem der Ansprüche 13 bis 18, **dadurch gekennzeichnet, dass** anatomische Patientenparameter wie Rasse, Alter, Geschlecht, Größe, Gewicht und/oder bisherige Behandlungen in einer Datenbank abgelegt sind und in die Berechung einfließen.

20. Anordnung nach einem der Ansprüche 13 bis 19, **dadurch gekennzeichnet, dass** statistische Daten, wie das Verhältnis der einzelnen anatomischen Größen zueinander in einer Datenbank abgelegt sind und in die Berechnung einfließen.

21. Anordnung nach einem der Ansprüche 13 bis 20, **dadurch gekennzeichnet, dass** die Anordnung ein PC ist, der durch Software gesteuert wird.

22. Anordnung nach einem der Ansprüche 13 bis 21, **dadurch gekennzeichnet, dass** zur Zuordnung von Informationen zu Objekten, insbesondere Zähnen, die in einer digitalisierte Röntgenaufnahme bestimmt sind, weiterhin vorhanden sind:
- ein Speicherbereich, in dem die Röntgenaufnahme abgelegt ist, wobei der Röntgenaufnahme Objektkennzeichnungsinformationen zugeordnet sind,
- ein zweiter Speicherbereich, in dem Informationen zu den Objekten abgelegt sind, wobei Referenzen zwischen den Objekten und den Objektkennzeichnungsinformationen gespeichert werden,
wobei die auf dem Ausgabegerät dargestellten Objekte auswählbar und die ausgewählten Objekte optisch hervorgehoben dargestellt sind, wobei die Objekte weiterhin auswählbar sind, um hinterlegte Informationen zuzuordnen oder abzufragen.

23. Anordnung nach Anspruch 22, **dadurch gekennzeichnet, dass** einzelne oder mehrere ausgewählte Objekte abgewählt werden können.

24. Anordnung nach Anspruch 23, **dadurch gekennzeichnet, dass** in Folge der Auswahl eines Objektes zu weiteren Informationen verzweigt wird, so weit sie vorhanden sind.

25. Anordnung nach einem der Ansprüche 22 bis 24, **dadurch gekennzeichnet, dass** die Referenzen in Form von Verknüpfungen (Links) entweder unmittelbar beim Objekt und/oder unmittelbar bei der Information und/oder separat verwaltet werden.

26. Anordnung nach einem oder mehreren der Ansprüche 22 bis 25, **dadurch gekennzeichnet, dass** das Ausgabegerät ein Bildschirm ist und die weiteren Informationen in einem sich automatisch öffnenden Anzeigebereich (Pop-Up-Fenster) dargestellt werden oder die weiteren Informationen zu einem neuen Bildschirmaufbau führen.

27. Anordnung nach einem oder mehreren der Ansprüche 22 bis 26, **dadurch gekennzeichnet, dass** die weiteren Informationen Diagnose- und/oder Behandlungsinformationen und/oder weitere insbesondere detaillierte Röntgenaufnahmen sind.

28. Anordnung nach einem oder mehreren der Ansprüche 22 bis 27, **gekennzeichnet durch** eine Schnittstelle zu einem Röntgengerät, wobei das Röntgengerät über die Schnittstelle die Informationen in Form von Daten zur Darstellung als Röntgenaufnahmen sendet, wobei diese Informationen in einem dritten Speicherbereich abgelegt werden und eine Referenz in einem vierten Speicherbereich zu einem Objekt angelegt wird.

29. Anordnung nach einem oder mehreren der Ansprüche 22 bis 28, **dadurch gekennzeichnet, dass** die Informationen über mehrere Ebenen hierarchisch angeordnet sein können.

30. Anordnung nach einem oder mehreren der Ansprüche 22 bis 29, **gekennzeichnet durch** Mittel, die es erlauben, dass die Objekte manuell **durch** Auswahl eines bestimmten Bereichs auf der Röntgenaufnahme bestimmt werden können.

31. Anordnung nach einem oder mehreren der Ansprüche 22 bis 30, **gekennzeichnet durch** die Funktionalität eines Datenbanksystems.

32. Anordnung nach einem oder mehreren der Ansprüche 22 bis 31, **gekennzeichnet durch** die Merkmale einer Anordnung zur Identifikation von Objekten, insbesondere Zähnen, auf einer digitalisierten Röntgenaufnahme nach einem oder mehreren der vorhergehenden Ansprüche.

## Claims

1. A method of identifying one or more objects, particularly teeth, in a digitized X-ray image, wherein the areas depicting the possible object(s) are identified using image-processing algorithms involving segmentation and/or edge detection of the X-ray image and, for more accurate identification, said areas are computionally linked with those parameters of the X-ray apparatus which were used for acquiring the X-ray image, the parameters taken into account being position data and/or trajectories and/or starting and stopping points of the X-ray apparatus.

2. A method as defined in claim 1, wherein the apparatus-specific parameters are linked with parameters of a non-patient-dependent tooth data bank, in order to obtain probable current geometrical positions within the areas depicting the possible object(s).

3. A method as defined in claim 1 or claim 2, wherein clustering of recognized areas is carried out prior to linking with the apparatus-specific parameters.

4. A method as defined in any one of claims 1 to 3, wherein, in addition, patient-specific parameters are computationally linked in order to achieve more accurate identification of the areas depicting one or more possible object(s).

5. A method as defined in any one of claims 1 to 4, wherein anatomical patient characteristics such as race, age, sex, size, weight, and/or treatment history are taken account of.

6. A method as defined in any one of claims 1 to 5, wherein proposals are made to the user concerning recognized objects, which can be interactively modified or confirmed.

7. A method as defined in any one of claims 1 to 6, wherein the information thus ascertained on the objects is stored separately in a data base, from which it can be fetched for subsequent implementation.

8. A method as defined in any one of claims 1 to 7, wherein the digitized X-ray image is displayed and that object is selected for which further information should be stored, and, furthermore, following determination of an object, data concerning the display of digital images are received from an X-ray apparatus which are automatically assigned to the selected object.

9. A method as defined in claim 8, wherein the selected object is provided with a graphical label, which is placed over the images as an overlay.

10. A method as defined in claim 8 and/or claim 9, wherein the information is in the form of graphical labels, which can be placed over the images as an overlay.

11. A method as defined in one or more of claims 8 to 10, wherein regions of the object can be determined to which information can be assigned.

12. A method as defined in one or more of claims 8 to 11, wherein pop-up menus relevant to the individual objects can be displayed.

13. A system for the identification of objects, particularly teeth, in a digitized X-ray image, comprising
- an input/output device for interactive control of the system, and
- a processing unit which has access to the digitized X-ray image, has access to apparatus-specific parameters of the X-ray apparatus, and determines, by means of image-processing algorithms based on these parameters and involving segmentation and/or edge detection, those areas of the digitized X-ray image which depict the object, the apparatus-specific parameters which are taken into account being position data and/or trajectories and/or starting and stopping points of the X-ray apparatus.

14. A system as defined in claim 13, wherein said processing unit has access to a non-patient-dependent tooth data bank.

15. A system as defined in claim 13 or claim 14, wherein said processing unit has means for clustering the areas identified by segmentation and/or edge detection.

16. A system as defined in any one of claims 13 to 15, wherein said processing unit has access to patient-specific information.

17. A system as defined in any one of claims 13 to 16, wherein means are provided for offering the user a proposal, which can be interactively confirmed, rejected, or modified.

18. A system as defined in any one of claims 13 to 17, **characterized by** a computer interface for the X-ray apparatus, via which the apparatus-specific data can be accessed.

19. A system as defined in any one of claims 13 to 18, wherein anatomical patient characteristics such as race, age, sex, size, weight, and/or treatment history are stored in a data base and are taken account of.

20. A system as defined in any one of claims 13 to 19, wherein statistical data, such as the relationship of the individual anatomical sizes to each other, are stored in a data base and are taken account of.

21. A system as defined in any one of claims 13 to 20, wherein the system is a software controlled PC.

22. A system as defined in any one of claims 13 to 21, wherein for the purpose of assigning information to objects, particularly teeth, identified in a digitized X-ray image, the following are also present:
- a memory area, in which the X-ray image is stored together with relevant object-labeling information,
- a second memory area, in which information concerning the objects is stored together with references linking said objects to said object-labeling information,
the objects displayed on the output device being selectable, while the selected objects are high-lighted and can be further selected to assign stored information thereto or to retrieve such information.

23. A system as defined in claim 22, wherein individually or severally selected objects can be discarded.

24. A system as defined in claim 23, wherein, following the selection of an object, links to further information can be followed, if present.

25. A system as defined in any one of claims 22 to 24, wherein said references are in the form of links and are administered either directly with the object and/or directly with the information and/or separately.

26. A system as defined in one or more of claims 22 to 25, wherein the output device is a video monitor and the other information is displayed in an automatically opening display area (pop-up window), or said further information causes a new screen build-up.

27. A system as defined in one or more of claims 22 to 26, wherein said further information consists of diagnostic and/or treatment information and/or other more detailed X-ray images.

28. A system as defined in one or more of claims 22 to 27, **characterized by** a computer interface for an X-ray apparatus, which X-ray apparatus sends, via said computer interface, information in the form of data for display thereof as an X-ray image, which information is stored in a third memory area, and a reference is linked to an object in a fourth memory area.

29. A system as defined in one or more of claims 22 to 28, wherein the information can be arranged hierarchically at a number of levels.

30. A system as defined in one or more of claims 22 to 29, **characterized by** means that allow the objects to be manually identified by selection of a certain area of the X-ray image.

31. A system as defined in one or more of claims 22 to 30, **characterized by** the functionality of a data bank system.

32. A system as defined in one or more of claims 22 to 31, **characterized by** the features of a system for identification of objects, particularly teeth, in a digitized X-ray image as defined in one or more of the previous claims.

## Revendications

1. Procédé pour identifier un ou plusieurs objets, notamment des dents sur un enregistrement radiographique numérisé, les zones représentant le ou les objets possibles étant déterminées avec des algorithmes de traitement d'image par segmentation et/ou détection des bords de l'enregistrement radiographique, **caractérisé en ce que** ces zones sont combinées mathématiquement entre elles pour la suite de la détermination des paramètres de l'appareil de radiographie utilisés pour réaliser l'enregistrement radiographique, les paramètres intervenant dans le calcul étant les données de position et/ou les portions de trajectoire et/ou les points de départ et d'arrêt de l'appareil de radiographie.

2. Procédé selon la revendication 1, **caractérisé en ce que** les paramètres spécifiques à l'appareil sont combinés avec des paramètres issus d'une base de données dentaire indépendante du patient afin d'obtenir les positions géométriques actuelles probables sur les zones représentant le ou les objets possibles.

3. Procédé selon la revendication 1 ou 2, **caractérisé en ce qu'**un regroupement des zones détectées (formation de cluster) est réalisé avant la combinaison avec les paramètres spécifiques à l'appareil.

4. Procédé selon l'une des revendications 1 à 3, **caractérisé en ce que** des paramètres supplémentaires spécifiques au patient sont combinés mathématiquement pour la suite de la détermination des zones représentant le ou les objets possibles.

5. Procédé selon l'une des revendications 1 à 4, **caractérisé en ce que** les paramètres anatomiques du patient tels que la race, l'âge, le sexe, la taille, le poids et/ou les traitements précédent interviennent dans le calcul.

6. Procédé selon l'une des revendications 1 à 5, **caractérisé en ce que** des proposition sur les objets détectés sont faites à l'utilisateur, lesquelles peuvent être adaptées ou confirmée de manière interactive.

7. Procédé selon l'une des revendications 1 à 6, **caractérisé en ce que** les informations ainsi déterminées des objets sont enregistrées séparément dans une base de données et peuvent être invoquées pour une nouvelle utilisation.

8. Procédé selon l'une des revendications 1 à 7, **caractérisé en ce que** l'image radiographique numérisée est représentée et que l'objet pour lequel il faut enregistrer des informations supplémentaires est sélectionné et qu'ensuite des données destinées à représenter des images numériques sont reçues de la part d'un appareil de radiographie après la détermination de l'objet, lesquelles sont automatiquement associées à l'objet sélectionné.

9. Procédé selon la revendication 8, **caractérisé en ce que** l'objet sélectionné est muni d'un marquage graphique qui est placé en tant que couche superposée au-dessus des enregistrements.

10. Procédé selon une ou plusieurs des revendications 8 à 9, **caractérisé en ce que** les informations sont des marquages graphiques qui peuvent être placés en tant que couches superposées au-dessus des enregistrements.

11. Procédé selon une ou plusieurs des revendications 8 à 10, **caractérisé en ce qu'**il est possible de définir les zones de l'objet dans lesquelles peuvent être associées des informations.

12. Procédé selon une ou plusieurs des revendications 8 à 11, **caractérisé en ce qu'**il est possible d'invoquer des menus contextuels à propos de chacun des objets.

13. Arrangement pour identifier des objets, notamment des dents sur un enregistrement radiographique numérisé,
- comprenant un appareil d'entrée et de sortie pour la commande interactive de l'arrangement,
- comprenant une unité de traitement qui a accès à l'enregistrement radiographique numérisé, qui a accès aux paramètres spécifiques de l'appareil de radiographie et comprenant des moyens pour déterminer les zones représentant le ou les objets possibles sur la base de ces informations et pour segmenter et/ou détecter les bords sur l'enregistrement radiographique numérisé, les paramètres spécifiques de l'appareil invoqués pour le calcul étant les données de position et/ou les portions de trajectoire et/ou les points de départ et d'arrêt de l'appareil de radiographie.

14. Arrangement selon la revendication 13, **caractérisé en ce que** l'unité de traitement a accès à une base de données indépendante du patient.

15. Arrangement selon la revendication 13 ou 14, **caractérisé en ce que** l'unité de traitement présente des moyens pour former un cluster des zones présentes après la segmentation et/ou la détection des bords.

16. Arrangement selon l'une des revendications 13 à 15, **caractérisé en ce que** l'unité de traitement a accès à des informations spécifiques au patient.

17. Arrangement selon l'une des revendications 13 à 16, **caractérisé en ce que** des moyens sont prévus pour soumettre à l'utilisateur une proposition qu'il peut accepter, refuser ou modifier de manière interactive.

18. Arrangement selon l'une des revendications 13 à 17, **caractérisé par** une interface vers l'appareil de radiographie par le biais de laquelle il est possible d'accéder aux données spécifiques à l'appareil.

19. Arrangement selon l'une des revendications 13 à 18, **caractérisé en ce que** des paramètres anatomiques du patient tels que la race, l'âge, le sexe, la taille, le poids et/ou les traitements précédent sont enregistrés dans une base de données et interviennent dans le calcul.

20. Arrangement selon l'une des revendications 13 à 19, **caractérisé en ce que** des données statistiques telles que le rapport entre les différentes grandeurs anatomiques entre elles sont enregistrées dans une base de données et interviennent dans le calcul.

21. Arrangement selon l'une des revendications 13 à 20, **caractérisé en ce que** l'arrangement est un PC qui est commandé par des logiciels.

22. Arrangement selon l'une des revendications 13 à 21, **caractérisé en ce que** les éléments suivants sont également présents pour l'association des informations aux objets, notamment aux dents, qui sont déterminées dans un enregistrement radiographique numérisé :
- une zone de mémoire dans laquelle est stocké l'enregistrement radiographique, des informations d'identification de l'objet étant associées à l'enregistrement radiographique,
- une deuxième zone de mémoire dans laquelle sont stockées des informations sur les objets, des références entre les objets et les informations d'identification d'objet étant enregistrées,
les objets représentés sur l'appareil de sortie pouvant être sélectionnés et les objets sélectionnés étant mis en valeur visuellement, les objets pouvant toujours être sélectionnés pour associer ou interroger les informations sous-jacentes.

23. Arrangement selon la revendication 22, **caractérisé en ce qu'**il est possible de sélectionner des objets sélectionnés individuels ou plusieurs objets sélectionnés.

24. Arrangement selon la revendication 23, **caractérisé en ce qu'**à la suite de la sélection d'un objet, on bascule sur des informations supplémentaires, sous réserve qu'elles soient présentes.

25. Arrangement selon l'une des revendications 22 à 24, **caractérisé en ce que** les références sont gérées sous la forme de liens (links) soit directement au niveau de l'objet et/ou directement au niveau de l'information et/ou séparément.

26. Arrangement selon une ou plusieurs des revendications 22 à 25, **caractérisé en ce que** l'appareil de sortie est un écran et les informations supplémentaires sont représentées dans une zone d'affichage (fenêtre incrustée) qui s'ouvre automatiquement ou alors les informations supplémentaires donnent lieu à une nouvelle disposition à l'écran.

27. Arrangement selon une ou plusieurs des revendications 22 à 26, **caractérisé en ce que** les informations supplémentaires sont des informations de diagnostic et/ou de traitement et/ou des enregistrements radiographiques supplémentaires particulièrement détaillés.

28. Arrangement selon une ou plusieurs des revendications 22 à 27, **caractérisé par** une interface vers un appareil de radiographie, l'appareil de radiographie envoyant par le biais de l'interface les informations sous la forme de données en vue de leur représentation sous la forme d'enregistrements radiographiques, ces informations étant stockées dans une troisième zone de mémoire et une référence vers un objet étant créée dans une quatrième zone de mémoire.

29. Arrangement selon une ou plusieurs des revendications 22 à 28, **caractérisé en ce que** les informations peuvent être disposées hiérarchiquement sur plusieurs plans.

30. Arrangement selon une ou plusieurs des revendications 22 à 29, **caractérisé par** des moyens qui permettent que les objets peuvent être définis manuellement en sélectionnant une zone spécifique sur l'enregistrement radiographique.

31. Arrangement selon une ou plusieurs des revendications 22 à 30, **caractérisé par** la fonctionnalité d'un système de base de données.

32. Arrangement selon une ou plusieurs des revendications 22 à 31, **caractérisé par** les caractéristiques d'un arrangement pour identifier des objets, notamment des dents sur un enregistrement radiographique numérisé selon une ou plusieurs des revendications précédentes.
